(19) Europäisches Patentamt
European Patent Office
Office européen des brevets

(11) **EP 3 260 156 A1**

(12) **EUROPEAN PATENT APPLICATION**

(43) Date of publication:
27.12.2017 Bulletin 2017/52

(51) Int Cl.:
*A61M 25/01* (2006.01)       *A61B 5/0215* (2006.01)
*A61B 5/00* (2006.01)

(21) Application number: 17176629.8

(22) Date of filing: 14.09.2010

(84) Designated Contracting States:
AL AT BE BG CH CY CZ DE DK EE ES FI FR GB
GR HR HU IE IS IT LI LT LU LV MC MK MT NL NO
PL PT RO SE SI SK SM TR

(30) Priority: 15.09.2009 SE 0950671
15.09.2009 US 242502 P

(62) Document number(s) of the earlier application(s) in
accordance with Art. 76 EPC:
15202854.4 / 3 020 441
10817517.5 / 2 477 685

(71) Applicant: St. Jude Medical Coordination Center
BVBA
1930 Zaventem (BE)

(72) Inventor: SMITH, Leif
Deceased (SE)

(74) Representative: Brann AB
P.O. Box 3690
Drottninggatan 27
103 59 Stockholm (SE)

Remarks:
•This application was filed on 19.06.2017 as a
divisional application to the application mentioned
under INID code 62.
•Claims filed after the date of filing of the application
/ after the date of receipt of the divisional application
(Rule 68(4) EPC).

(54) **RAPID EXCHANGE GUIDE UNIT**

(57)    Rapid exchange guide unit comprising an elongated support member 3, and a guide wire member (11) provided with a guide wire lumen (13) having a distal guide wire opening (15) and a proximal guide wire opening (17), the guide wire lumen is arranged close to the distal end of said elongated support member, and is adapted to receive a guide wire. The rapid exchange guide unit further comprises at least one sensor (19) arranged close to the distal end of the elongated support member, and being adapted to measure a parameter in a living body, and to generate a sensor signal in dependence of the measured parameter. The sensor signal is applied to a signal processing unit adapted to process the sensor signal and to generate a processed sensor signal.

FIG. 3a

## Description

Field of the invention

The present invention relates to a rapid exchange guide unit according to the preamble of the independent claim.

Generally, a so called rapid exchange catheter includes features that allows for easy exchange of the guide unit without the removal of the guide wire.

This invention relates to a rapid exchange guide unit with multiple utilities for use inside mammalian tubular vessels or structures, and more particularly allows the guide unit to be removed from around a guide wire by using a slot or channel to hold the guide wire.

Background of the invention

During catheter-based procedures, the physician often visualizes the area being treated under fluoroscopy and visualizes the catheter and/or treatment area using radio-opaque materials. Contrast dyes are used to visualize the treated area by injecting a contrast dye through the catheter while the fluoroscope is being operated. The physician can then see the vessel in which the catheter is positioned, as well as any lesion past which the contrast dye flows.

In the procedure, the physician may use a guiding device, such as a guide wire, to controllably reach the lesion or area to be treated. Once the guide wire is in position, the physician may need to pass one or more catheters, tubular devices, and/or medical devices along the guide wire to the lesion or treatment area. The physician may pull the catheter or tubular device back along the guide wire and finally off of the guide wire. A difficulty of this, however, is that the guide wire must be very long (i.e. longer than the catheter) in order to pull the catheter off the guide wire without needing to first also pull the guide wire out of the patient. A known solution to this problem is the use of a rapid exchange configuration in which the distal end of the catheter has a pair of openings into a lumen and through which the guide wire may be passed by inserting the proximal end of the guide wire through the distal most opening and then passing the proximal end of the guide wire out of the proximal opening of the lumen. For example, such a configuration is described in U-5,451,233.

Also WO/2003/039626 relates to a rapid exchange catheter with stent deployment, therapeutic infusion, and lesion sampling features.

One common application of rapid exchange and marker catheters is during coronary angioplasty, which refers to the use of an inflatable balloon to increase the blood flow through a stenosis (i.e. a partially blocked section of a blood vessel feeding the heart). A typical coronary angioplasty consists of three steps. First, a physician inserts a guiding catheter into a patient's blood vessel, typically through the femoral artery at the top of the patient's leg. The guiding catheter is advanced toward the heart through the patient's blood vessel, stopping short of the coronary arteries, and is then fixed in place. Next, the physician inserts a guide wire into the guiding catheter until the distal end of the guide wire exits the guiding catheter and enters the coronary artery. The physician then positions the guide wire across the stenosis to be treated in the coronary artery, and the guide wire is fixed in place.

Finally, the physician advances a balloon catheter along the guide wire until the balloon exits the guiding catheter and is positioned across the stenosis. The physician then inflates the balloon to treat the stenosis, deflates the balloon, and removes the balloon catheter without disturbing the placement of either the guide wire or the guiding catheter.

Physicians frequently need to exchange balloon catheters during a single coronary angioplasty procedure. For example, if a stenosis blocks most of the blood flow through a vessel, the physician may first need to use a small balloon to increase the size of the opening through the stenosis, and then use a larger balloon to further increase the opening. Another example of a catheter exchange is when a physician uses a first balloon catheter to open a lumen and a second catheter to deploy a stent.

Catheters are used in a variety of minimally invasive medical procedures. A major portion of the catheter field involves catheters that track over a guide wire, such as angioplasty catheters that are used to advance an inflatable member over a guide wire to a desired vascular location. One advancement in this field has been the use of rapid exchange catheters in place of standard over-the-wire catheters.

A standard over-the-wire catheter typically tracks over a guide wire over its entire length such that, in order to maintain a distal guide wire location while exchanging the catheter, a guide wire extension or very long guide wire is used. To exchange the standard over-the-wire catheter, the guide wire is held in place while the catheter is withdrawn. The proximal end of the guide wire is held until the distal end of the catheter exits the patient's body, while the distal end of the guide wire remains in the desired location, meaning that the guide wire, during exchange, must be twice as long as the catheter.

A rapid exchange catheter tracks over the guide wire for only a short distal portion of the catheter. Examples of rapid exchange catheters, their use, and methods for making such catheters are illustrated by US-6,409,863. The catheter shown by the US-patent includes an outer member and a distally located inner member, with a balloon proximal end attached to the distal end of the outer member and a balloon distal end attached to the distal inner member. A proximal guide wire port is located distal of the proximal end of the catheter, with the distal inner member opening at its proximal end to the proximal guide wire port, and extending to the distal end of the catheter.

According to the general procedure when determining vessel constrictions first a conventional guide wire is inserted and guided into e.g. a coronary vessel to be investigated. Then a catheter, preferably a so called rapid exchange catheter, is threaded over the guide wire and inserted and guided by the guide wire into the measurement site in the coronary vessel. Contrast fluid is then inserted, via the catheter, into the measurement site in the vessel. By viewing the site using angiography, the physician visually determines the significance of the constriction, and whether e.g. a stent placement or ballon expansion needs to be performed.

An alternative to using visual determination of the significance of a constriction is using measurement of fractional flow reserve (FFR). FFR is defined as the ratio of distal (to stenosis) pressure (Pd) to aortic pressure (Pa) during hyperemia. US-6,565,514, assigned to the same assignee as the present application, discloses a method and system for determining physiological variables such as arterial blood pressure. For determining the so called Myocordial Fractional Flow Reserve, $FFR_{myo}$, two pressures must be measured, namely the arterial pressure before a stenosis, and the pressure distally of the stensosis. $FFR_{myo}$ is defined as maximum myocordial flow in the presence of a stenosis in the supplying epicardial coronary artery, divided by normal maximum flow. $FFR_{myo}$ is calculated by means of the formula:

$$FFR_{myo} = (P_d - P_v)/(P_a - P_v) = P_d / P_a,$$

wherein

- $P_d$ =arterial pressure at maximum hyperemia;
- $P_a$ =distal coronary pressure at maximum hyperemia;
- $P_v$ =central venous pressure at maximum hyperemia.

It is a lesion-specific index of the functional severity of the stenosis and can be obtained by intracoronary pressure measurement by a guide wire-mounted pressure sensor. During PTCA, balloon angioplasty or Percutaneaus Transluminal Coronary Angioplasty, the separate contributions of coronary and collateral blood flow to maximum myocardial perfusion can be obtained.

Thus, an object of the device of US-6,565,514, is to provide improved systems for monitoring physiological variables, in particular for pressure measurements in the coronary vessels, and especially for the reliable determination of Fractional Flow Reserve, $FFR_{myo}$.

Thus, if a constriction should be further investigated it is sometimes necessary to measure the pressure and flow in the vessel. The Fractional Flow Reserve (FFR) value may then be determined which gives a clear indication of the constriction, and in order to calculate the FFR the pressure values distally and proximally the constriction are required.

In the procedure used today, the guide wire is then either replaced by a guide wire provided with a pressure sensor at the distal end and the pressure measurements are then performed, or a guide wire provided with a pressure sensor at its distal end is inserted via another lumen of the catheter into the site of interest. This is often regarded as a rather lengthy and complicated procedure and the object of the present invention is to improve the procedure and the devices used today.

More generally, the object of the present invention is to provide an improved rapid exhange procedure which also facilitates measurements of physiological variables and other variables inside the vessel.

## Summary of the invention

The above-mentioned object is achieved by the present invention according to the independent claim.

Preferred embodiments are set forth in the dependent claims.

According to the present invention a sensor is arranged at the distal end of the rapid exchange guide unit. The sensor is adapted to measure a parameter in a living body, and to generate a sensor signal in dependence of the measured parameter. The sensor signal is applied to a signal processing unit adapted to process the sensor signal and to generate a processed sensor signal. The guide wire member has further a longitudinal extension of 1-5 cm, and the inner diameter of the guide wire lumen is less than 2 mm.

According to one preferred embodiment, the sensor is a pressure sensor, which pressure sensor comprises a sensor support body (a "sensor chip") provided with a diaphragm covering a cavity formed in the support body having a pressure sensitive element mounted on the diaphragm, for recording pressure. The pressure sensitive element is preferably a piezoresistive element. A pressure sensor applicable in connection with the present invention is disclosed in US-6,615,667, assigned to the assignee in the present application. This known sensor has an exemplary geometrical extension of 0,18 mm x 1,3 mm x 0,18 mm.

According to another embodiment of the present invention the guide unit comprises an elongated guide member being in the form of a catheter, essentially being a hollow tube, and is provided with a sensor at its distal end adapted to perform measurements. Thereby the measurements may be performed directly and as a consequence no additional measurement guide wire has to be inserted. In addition the measurements are performed at exactly the correct position in that no positioning is required as it is when inserting a dedicated guide wire.

Furthermore, the measurements may be performed essentially at the same time as the fluid contrast is inserted and expelled from the distal opening of the catheter, thereby saving time.

According to the present invention an improved rapid exchange guide unit is achieved that enables more accurate, less expensive measurement procedures to be performed, that in addition facilitates the physician to perform the angioplasty, i.e. restoration of normal blood flow, by laser surgery or balloon expansion, at an even higher grade of accuracy with regard to position of the constriction.

Short description of the appended drawings

Figure 1a shows a schematic side view of the rapid exchange guide unit according to the present invention.

Figure 1b shows a schematic side view of the rapid exchange guide unit provided with two sensors according to an embodiment of the present invention.

Figure 2a shows a schematic side view of the rapid exchange guide unit according to a first embodiment.

Figure 2b shows a schematic side view of the rapid exchange guide unit provided with two sensors according to a first embodiment.

Figure 3a shows a schematic side view of the rapid exchange guide unit according to a second embodiment.

Figure 3b shows a schematic side view of the rapid exchange guide unit provided with two sensors according to a second embodiment.

Figure 4 shows a schematic block diagram illustrating the functional parts of the present invention.

Figures 5-8 show schematic side views of the catheter wall illustrating different arrangements of a pressure sensor in the wall.

Detailed description of preferred embodiments of the invention

The present invention will now be described in detail with references to the appended drawings. The drawings illustrate the schematic structure of different embodiments, and are not in a correct scale, e.g. with regard to the size of the sensor in relation to the elongated guide member.

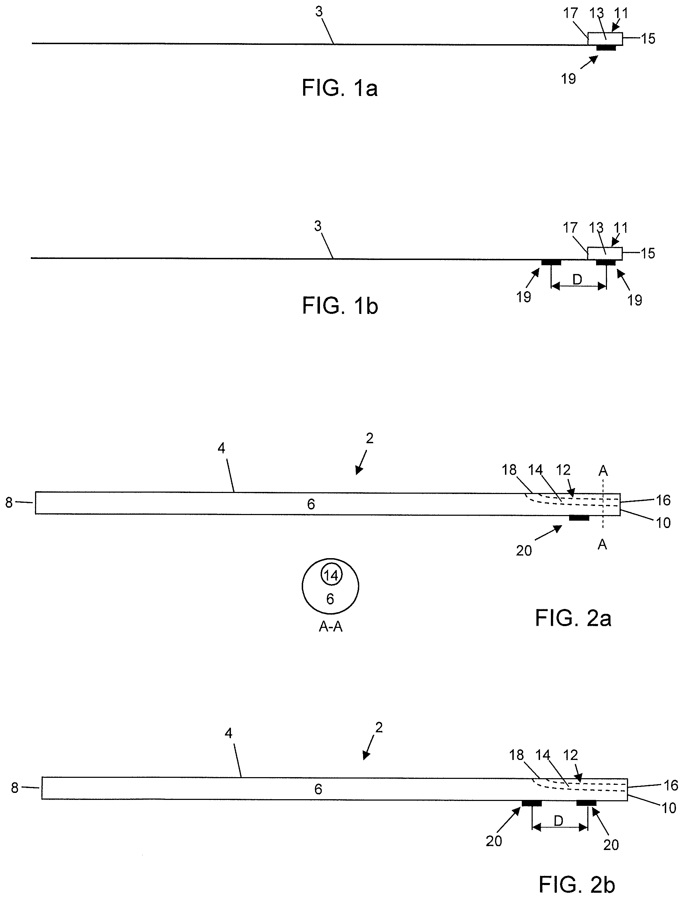

Figure 1a shows a schematic side view of the rapid exchange guide unit according to the present invention. The rapid exchange guide unit comprises an elongated support member 3 and a guide wire member 11 provided with a guide wire lumen 13 having a distal guide wire opening 15, and a proximal guide wire opening 17, the guide wire lumen is arranged close to the distal end of said elongated support member, and is adapted to receive a guide wire. The distance between the proximal guide wire opening 17 and the distal end of the elongated support member 3 is in the range of 1-5 cm.

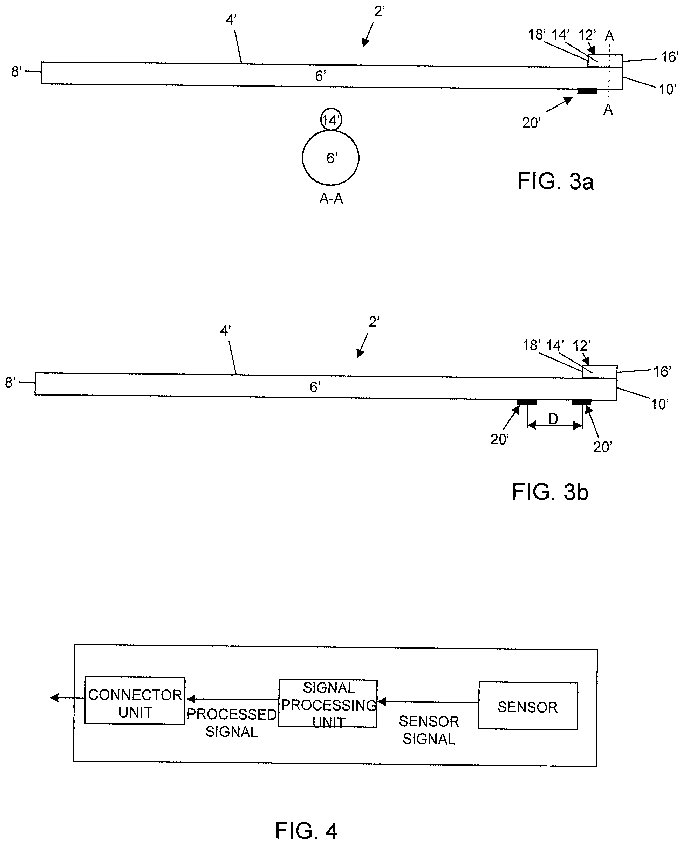

The rapid exchange guide unit further comprises at least one sensor 19 arranged close to the distal end of the elongated support member, and being adapted to measure a parameter in a living body, and to generate a sensor signal in dependence of the measured parameter. The generated sensor signal is applied to a signal processing unit (see figure 4) adapted to process the sensor signal and to generate a processed sensor signal. The measured parameter may be a physiological variable, e.g. pressure, temperature, or flow, or a physical variable, e.g. electromagnetic waves or radio waves. Thus, according to one embodiment the sensor is used to sense physical parameters, e.g. electromagnetic waves or radio waves. This embodiment is applicable in situations when the position of the sensor is to be determined. One or many radio wave signals is then generated from outside the body and from different directions and the position of the sensor may then be determined by analysing reflected signals. The sensor is e.g. a frequency tuned circuit.

The elongated support member may be in the form of a wire, or in the form of a thin metal tubing. The support member may also be in the form of a combination of a wire and a metal tubing. The elongated support member, according to this embodiment, has the advantage of having a thin structure along the major part of its length, it is only at its distal end where the guide wire lumen is arranged that the width is slightly increased.

The guide unit further comprises a connector unit arranged at the proximal end of the catheter for attachment to an external device. The connector unit provides for electrical connection to the signal processing unit and to the sensor.

In an alternative embodiment the signal processing unit is instead arranged in a proximal part of the guide unit or at an external device to which the guide unit is attached. According to this alternative embodiment the raw unprocessed sensor signal is supplied by the electrical cable(s) along the guide member to the signal processing unit.

In one embodiment the signal processing unit comprises a Wheatstone bridge, or any equivalent circuitry adapted to filter, amplify and process the measured sensor signal.

According to an alternative embodiment the processed sensor signal, or the unprocessed sensor signal, may be wirelessly transferred to an external device, e.g. an external monitor (not shown).

According to one preferred embodiment of the present invention, the sensor is adapted to measure pressure. The pressure sensor then comprises a sensor support body with a maximal geometrical extension of 1,5 mm and is provided with a diaphragm covering a cavity formed in the support body having a pressure sensitive element mounted on the diaphragm, for recording pressure. According to this preferred embodiment, the pressure sensitive element is a piezoresistive, piezocapacitive or a piezoelectric element.

However, as an obvious constructional variation, the sensor may instead or in combination with measuring pressure, be adapted to measure one or many of temperature, flow, and position.

In Figure 1b, another preferred embodiment of the present invention is shown, wherein two sensors 19 are arranged at a predetermined distance D from each other in the longitudinal direction of the elongated guide member 3. The predetermined distance D may be such that, when in use, the proximal sensor senses a reference parameter in relation to the parameter sensed by the distal sensor. The predetermined distance D is approximately 5 - 20 mm. The distance D between the two sensors is preferably chosen such that when one sensor is arranged proximally a suspected stenosis, the other sensor will then be arranged distally the stenosis.

In a further embodiment of the present invention, the guide unit comprises an elongated guide member being in the form of a guide wire or catheter, provided with a sensor comprising a magnetic detection probe, for detecting a plurality of magnetic fields, and being part of a medical positioning system, such as that described in US 6,233,476 and US 2004/0097804.

With reference to figures 2a and 3a, showing schematic side views of the rapid exchange guide unit according to a second and a third embodiment of the invention will now be described in detail. In the lower part in each of the figures 2a and 3a a cross-sectional view along A-A is shown.

In the rapid exchange guide unit 2, 2' according to the second and third embodiments the elongated guide member 3 is a catheter member 4, 4' provided with a catheter lumen 6, 6' having a proximal catheter opening 8, 8' and a distal catheter opening 10, 10', preferably arranged to expel contrast fluid at a measurement site, and a guide wire member 12, 12' provided with a guide wire lumen 14, 14' having a distal guide wire opening 16 and a proximal guide wire opening 18, 18'. The guide wire lumen runs essentially parallel to the catheter lumen and is adapted to receive a guide wire.

Furthermore, the proximal guide wire opening is arranged at a location along the catheter member distally of the proximal catheter opening of the catheter member, and that the distal guide wire opening is arranged at a location close to the distal catheter opening.

The catheter further comprises at least one sensor 20, 20' arranged close to the distal end of the catheter. The sensor is adapted to measure a parameter in a living body, and to generate a sensor signal in dependence of the measured parameter. The sensor signal is applied to a signal processing unit (see figure 4), preferably arranged in connection with the sensor adapted to process the sensor signal and to generate a processed sensor signal (see figure 4). The catheter further comprises a connector unit arranged at the proximal end of the catheter for attachment to an external device. The connector unit provides for electrical connection, and also a fluid tight connection when e.g. a contrast fluid is to be supplied to the catheter.

In an alternative embodiment the signal processing unit is instead arranged in a proximal part of the catheter or at an external device to which the catheter is attached. According to this alternative embodiment the raw unprocessed sensor signal is supplied by the electrical cable(s) along the catheter to the signal processing unit.

According to a preferred embodiment of the present invention, the sensor is a pressure sensor. The pressure sensor comprises a sensor support body with a maximal geometrical extension of 1,5 mm and is provided with a diaphragm covering a cavity formed in the support body having a pressure sensitive element mounted on the diaphragm, for recording pressure. The pressure sensitive element is a piezoelectric, piezoresistive or piezocapacitive pressure element.

However, as an obvious constructional variation, the sensor may instead or in combination with measuring pressure, be adapted to measure one or many of temperature, flow, and position.

In one embodiment the signal processing unit comprises a Wheatstone bridge, or any equivalent circuitry adapted to filter, amplify and process the measured sensor signal.

According to an alternative embodiment the processed sensor signal, or the unprocessed sensor signal, may be wirelessly transferred to an external device, e.g. an external monitor (not shown).

In figures 2b and 3b further embodiments of the present invention are shown, wherein two sensors are arranged a predetermined distance D from each other in the longitudinal direction of the catheter. The predetermined distance D may be such that, when in use, the proximal sensor senses a reference parameter in relation to the parameter sensed by the distal sensor.

According to a preferred embodiment, the sensors are sensitive to pressure. The predetermined distance may then be such that, when in use, the proximal pressure sensor senses a reference pressure in relation to the pressure sensed by the distal pressure sensor, and the obtained pressure values may be used to determine Fractional Flow Reserve (FRR) values.

With reference to figure 2a and 2b another embodiment of the present invention is illustrated where the guide wire member 12 is arranged such the guide wire lumen 14 runs parallel to and within the catheter lumen 6. The proximal guide wire member opening 18 is arranged as an opening in the catheter member wall.

With reference to figure 3a and 3b one embodiment of the present invention is illustrated where the guide wire member 12' is arranged such the guide wire lumen 14' runs parallel to and outside the catheter lumen 6'.

In the disclosed embodiments the guide wire member 12, 12' has an essentially tubular extension having a circular cross-section, naturally other geometrical shapes are possible, e.g. elliptical, elongated etc.

Furthermore, the guide wire member is here disclosed as a closed tube but also a tube provided with a longitudinal slot, e.g. at the upper part of the guide wire member 12' in figure 3, would be possible, through which slot a guide wire is pressed into the guide wire lumen. In that case the guide wire member must have a structural shape integrity to regain its original shape but have enough flexibility to allow the slot to be widened.

For all embodiments the guide wire member has a longitudinal extension in the order of 1-5 cm.

Preferably, the inner diameter of the guide wire lumen is less than the inner diameter of the catheter lumen. However, for the embodiment illustrated in figure 3a and 3b the diameters of the catheter lumen 6' and guide wire lumen 14' may be equal or the guide wire lumen may even have the larger diameter.

The inner diameter of the guide wire lumen is less than 3 mm, preferably less than 2 mm.

Preferably, the proximal catheter opening 8 is provided with a contrast fluid connection port that in turn is connectable, by use of the connector unit, to an external device (not shown in the figures) adapted to apply contrast fluid to the catheter.

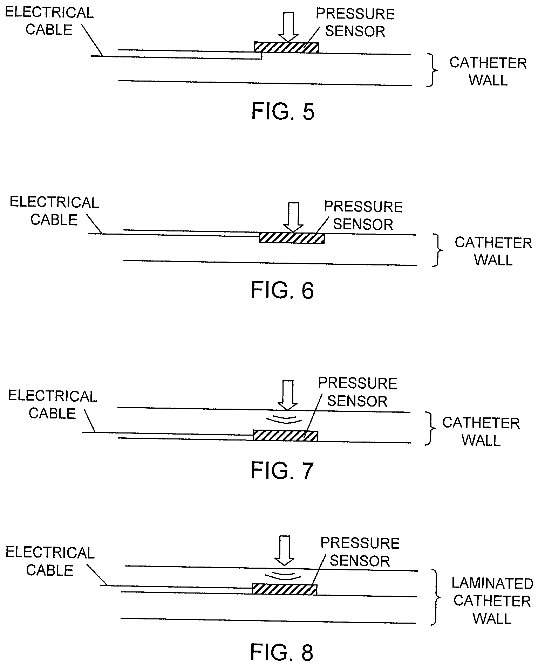

Figures 5-8 show schematic side views of the catheter wall illustrating different arrangements of the pressure sensor in the catheter wall of the catheter member or guide wire member. These different arrangements are applicable to any of the above described embodiments illustrated in figures 1-3.

According to one embodiment the at least one pressure sensor is arranged at an outer surface of the catheter member or guide wire member, this is illustrated by figure 5.

According to another embodiment the at least one pressure sensor is arranged in a recess in the outer surface of the catheter member or guide wire member, this is illustrated by figure 6.

According to another embodiment the at least one pressure sensor is arranged at an inner surface of the catheter member or guide wire member, this is illustrated by figure 7. In figure 7 the sensitive part of the sensor is facing the arrow indicating pressure to be sensed. It is also possible to turn around the sensor such that the sensitive part instead faces the inner of the catheter member or guide wire member.

According to another embodiment the at least one pressure sensor is arranged in a catheter member wall or guide wire member, this is illustrated by figure 8. In this embodiment the catheter wall preferably is laminated whereas a recess is arranged in one of the layers adapted to receive the pressure sensor.

As illustrated in figures 5-8 the catheter further comprises one or many electrical cables connected to the at least one pressure sensor and said signal processing unit and running along the catheter, the cables being embedded in the catheter wall and connected to a connector unit arranged at the proximal end of the catheter.

The arrangements shown in figures 5-8 are also applicable to other types of sensor, such as sensors adapted to measure one or many of temperature, flow, and position.

In a further embodiment, not illustrated in the figures, the guide wire or catheter of the present invention comprises three sensors, each sensor measuring one or many of temperature, flow, and position. In one such embodiment, two sensors may determine pressure, while the third determines position.

The following non-limiting examples provide further illustration of the present disclosure.

A rapid exchange guide unit may comprise an elongated support member (3,4,4') and a guide wire member (11,12,12') provided with a guide wire lumen (13,14,14') having a distal guide wire opening (15,16,16') and a proximal guide wire opening (17,18,18'), wherein the guide wire lumen is arranged close to the distal end of said elongated support member, and is adapted to receive a guide wire, said guide wire member having a longitudinal extension of 1-5 cm. The inner diameter of the guide wire lumen is less than 2 mm, wherein that the rapid exchange guide unit further comprises at least one sensor (19,20,20') arranged close to the distal end of the elongated support member, and is adapted to measure a parameter in a living body, and to generate a sensor signal in dependence of the measured parameter, wherein said sensor signal is applied to a signal processing unit adapted to process the sensor signal and to generate a processed sensor signal.

A rapid exchange guide unit may comprise a sensor (19, 20, 20') being a pressure sensor (19, 20, 20'), which comprises a sensor support body with a maximal geometrical extension of 1,5 mm provided with a diaphragm covering a cavity formed in the support body having a pressure sensitive element mounted on the diaphragm, for recording pressure, said pressure sensitive element being a piezoresistive, piezocapacitive or a piezoelectric element, wherein said pressure signal is applied to said signal processing unit adapted to process the pressure signal and to generate a processed pressure signal.

A rapid exchange guide may comprise an elongated support member (3) being in the form of a wire.

A rapid exchange guide unit may comprise an elongated support member (3) being in the form of a thin metal tubing.

A rapid exchange guide may comprise an elongated support member (3) being in the form of a combination of a wire and a metal tubing.

A rapid exchange guide may comprise an elongated support member being a catheter member (4, 4') provided with a catheter lumen (6, 6') having a proximal catheter opening (8, 8') and a distal catheter opening (10, 10'), wherein the guide wire lumen runs essentially parallel to the catheter lumen, and wherein the proximal guide wire opening is arranged at a location along the catheter member distally of the proximal catheter opening of the catheter member, and wherein the distal guide wire opening is arranged at a location close to the distal catheter opening. In such a rapid exchange guide unit the sensor may be arranged at an outer surface of the catheter member, or in a recess in the outer surface of the catheter member, or at an inner surface of the catheter member, or in a catheter member wall. As an alternative, the sensor may be arranged at an outer surface of the guide wire member, or in a recess in an outer surface of the guide wire member, or at an inner surface of the guide wire member, or in a guide wire member wall.

A rapid exchange guide unit may comprise two sensors being arranged a predetermined distance from each other in the longitudinal direction of the guide unit. In such a rapid exchange guide unit said predetermined distance may be such that, when in use, the proximal sensor senses a reference parameter in relation to the parameter sensed by the distal sensor. Further, in such a rapid exchange guide unit, said sensors may be pressure sensors, each sensing a pressure value, and that said obtained pressure values are used to determine Fractional Flow Reserve (FRR) values.

A rapid exchange guide unit may comprise electrical cables connected to said at least one sensor and said signal processing unit and running along the unit and connected to a connector unit arranged at the proximal end of the guide unit.

A rapid exchange guide comprising a catheter lumen may be arranged such the guide wire lumen runs parallel to and outside said catheter lumen.

A rapid exchange guide unit comprising a catheter lumen may be arranged such the guide wire lumen runs parallel to and within said catheter lumen. In such a rapid exchange guide unit, the proximal guide wire member opening may be arranged as an opening in the catheter member wall.

In a rapid exchange guide unit comprising a catheter lumen, the inner diameter of the guide wire lumen may be less than the inner diameter of the catheter lumen.

In a rapid exchange guide unit, the inner diameter of the guide wire lumen may be less than 1 mm.

In a rapid exchange guide unit the processed sensor signal may be wirelessly transferred to an external monitor.

In any rapid exchange guide unit described above, the measured parameter may be a physiological variable or other physical variable. In such a rapid exchange guide unit, the sensor may be adapted to measure one or many of temperature, flow, and position.

The present invention is not limited to the above-described preferred embodiments. Various alternatives, modifications and equivalents may be used. Therefore, the above embodiments should not be taken as limiting the scope of the invention, which is defined by the appending claims.

**Claims**

1. A rapid exchange guide unit (2') comprising:

   a catheter member (4') having a catheter lumen (6') having a proximal catheter opening (8') and a distal catheter opening (10');
   a guide wire member (12') having a guide wire lumen (14') having a proximal guide wire opening (18') and a distal guide wire opening (16'), the guide wire member (12') being located near a distal end of the catheter member (4') and being adapted to receive a guide wire, and an inner diameter of the guide wire lumen (14') being less than 3 mm;
   a sensor (20') located near the distal end of the catheter member (4'), the sensor (20') being adapted to measure a parameter in a living body, and to generate a sensor signal based on the measured parameter such that the sensor signal may be applied to a signal processing unit adapted to process the sensor signal and to generate a processed sensor signal;

   **characterized in that:**

   the proximal guide wire opening (18') is located at a location along the catheter member distal of the proximal catheter opening (8'), and the guide wire lumen (14') runs essentially parallel to the catheter lumen (6').

2. The rapid exchange guide unit (2') according to claim 1, wherein the distal guide wire opening (16') is located at a location close to the distal catheter opening (10').

3. The rapid exchange guide unit (2') according to claim 2, wherein the distal guide wire opening (16') is in the same plane as the distal catheter opening (10'), in a direction perpendicular to an axial direction of the rapid exchange guide unit (2').

4. The rapid exchange guide unit (2') according to claim 1, wherein an inner diameter of the guide wire lumen (12') is less than an inner diameter of the catheter lumen (6').

5. The rapid exchange guide unit (2') according to claim 1, wherein the sensor (20') is located at an outer surface of the catheter member (4').

6. The rapid exchange guide unit (2') according to claim 1, wherein the sensor (20') is located in a recess of an outer surface of the catheter member (4').

7. The rapid exchange guide unit (2') according to claim 1, wherein the sensor (20') is located at an inner surface of the catheter member (4').

8. The rapid exchange guide unit (2') according to claim 1, wherein the sensor (20') is located in a wall of the catheter member.

9. The rapid exchange guide unit (2') according to claim 1, wherein the sensor (20') is a pressure sensor.

10. A rapid exchange guide unit (2) comprising:

a catheter member (4) having a catheter lumen (6) having a proximal catheter opening (8) and a distal catheter opening (10);
a guide wire member (12) having a guide wire lumen (14) having a proximal guide wire opening (18) and a distal guide wire opening (16), the guide wire member (12) being located near a distal end of the catheter member (4) and being adapted to receive a guide wire, and an inner diameter of the guide wire lumen (14) being less than 3 mm;
a sensor (20) located near a distal end of the catheter member (4), the sensor (20) being adapted to measure a parameter in a living body, and to generate a sensor signal based on the measured parameter such that the sensor signal may be applied to a signal processing unit adapted to process the sensor signal and to generate a processed sensor signal;

**characterized in that:**

the proximal guide wire opening (18) is located at a location along the catheter member distal of the proximal catheter opening (8), and
the guide wire lumen (14) runs within the catheter lumen (6).

11. The rapid exchange guide unit (2) according to claim 10, wherein the distal guide wire opening (16) is located at a location close to the distal catheter opening (10).

12. The rapid exchange guide unit (2) according to claim 11, wherein the distal guide wire opening (16) is in the same plane as the distal catheter opening (10), in a direction perpendicular to an axial direction of the rapid exchange guide unit (2).

13. The rapid exchange guide unit (2) according to claim 10, wherein the sensor (20) is located at an outer surface of the catheter member (4).

14. The rapid exchange guide unit (2) according to claim 10, wherein the sensor (20) is located in a recess of an outer surface of the catheter member (4).

15. A rapid exchange guide unit (2') comprising:

a catheter member (4, 4') having a catheter lumen (6, 6') having a proximal catheter opening (8, 8') and a distal catheter opening (10, 10');
a guide wire member (12, 12') having a guide wire lumen (14, 14') having a proximal guide wire opening (18, 18') and a distal guide wire opening (16, 16'), the guide wire member (12, 12') being located near a distal end of the catheter member (4, 4') and being adapted to receive a guide wire, and an inner diameter of the guide wire lumen (14, 14') being less than 3 mm;
a first sensor (20, 20') located near a distal end of the catheter member (4, 4'), the first sensor (20, 20') being adapted to measure a parameter in a living body, and to generate a sensor signal based on the measured parameter such that the sensor signal may be applied to a signal processing unit adapted to process the sensor signal and to generate a processed sensor signal;

**characterized in that:**

the first sensor (20, 20') is located at a location of the catheter member (4, 4') adjacent to the guide wire member (12, 12')
the rapid exchange guide unit further comprises a second sensor (20, 20') adapted to measure a parameter in a living body, and to generate a sensor signal based on the measured parameter such that the sensor signal may be applied to a signal processing unit adapted to process the sensor signal and to generate a processed sensor signal, the second sensor being located at a location of the catheter member (4, 4') proximal of the guide wire member (12, 12').

FIG. 1a

FIG. 1b

FIG. 2a

A-A

FIG. 2b

FIG. 3a

FIG. 3b

FIG. 4

ELECTRICAL CABLE    PRESSURE SENSOR    CATHETER WALL

**FIG. 5**

ELECTRICAL CABLE    PRESSURE SENSOR    CATHETER WALL

**FIG. 6**

ELECTRICAL CABLE    PRESSURE SENSOR    CATHETER WALL

**FIG. 7**

ELECTRICAL CABLE    PRESSURE SENSOR    LAMINATED CATHETER WALL

**FIG. 8**

Europäisches
Patentamt
European
Patent Office
Office européen
des brevets

# EUROPEAN SEARCH REPORT

Application Number

EP 17 17 6629

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 142 987 A (TSUGITA ROSS S [US]) 7 November 2000 (2000-11-07) * column 5, line 48 - column 6, line 22; claims; figures * | 1-15 | INV. A61M25/01 A61B5/0215 A61B5/00 |
| X | US 2007/083193 A1 (WERNETH RANDELL L [US] ET AL) 12 April 2007 (2007-04-12) * paragraphs [0118] - [0119], [0127]; claims; figures * | 1-6,8-15 | |
| X | US 5 046 497 A (MILLAR HUNTLY D [US]) 10 September 1991 (1991-09-10) * column 3, lines 24-37 * * column 6, line 35 - column 13, line 50; claims; figures * | 1-9,15 | |
| X | US 2006/167398 A1 (SOLAR RONALD J [US] ET AL) 27 July 2006 (2006-07-27) * paragraphs [0032], [0035]; claims; figures * | 1-15 | |
| X | US 2004/193021 A1 (ZDEBLICK MARK [US] ET AL) 30 September 2004 (2004-09-30) * paragraphs [0042] - [0057], [0067] - [0071]; claims; figures * | 1-15 | TECHNICAL FIELDS SEARCHED (IPC) A61M A61B |
| X | US 4 928 693 A (GOODIN RICHARD L [US] ET AL) 29 May 1990 (1990-05-29) * column 3, line 10 - column 4, line 29; claims; figures * | 1-15 | |
| X | US 2005/283221 A1 (MANN JAMES W [US] ET AL) 22 December 2005 (2005-12-22) * paragraphs [0028] - [0033], [0036] - [0037], [0040]; figures * | 1-15 | |

-/--

The present search report has been drawn up for all claims

| Place of search | Date of completion of the search | Examiner |
|---|---|---|
| The Hague | 6 November 2017 | Crisan, Carmen-Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/015541 A1 (ROSENBLUTH ROBERT F [US] ET AL) 17 January 2008 (2008-01-17) * paragraphs [0126] - [0127], [0139] - [0155]; claims; figures * ----- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 6 November 2017 | Crisan, Carmen-Clara |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**EP 3 260 156 A1**

## ANNEX TO THE EUROPEAN SEARCH REPORT
## ON EUROPEAN PATENT APPLICATION NO.

EP 17 17 6629

06-11-2017

| Patent document cited in search report | | Publication date | Patent family member(s) | | Publication date |
|---|---|---|---|---|---|
| US 6142987 | A | 07-11-2000 | AT | 342012 T | 15-11-2006 |
| | | | AT | 432670 T | 15-06-2009 |
| | | | CA | 2345654 A1 | 08-02-2001 |
| | | | DE | 1117343 T1 | 13-06-2002 |
| | | | DE | 60031234 T2 | 30-08-2007 |
| | | | EP | 1117343 A1 | 25-07-2001 |
| | | | EP | 1736115 A2 | 27-12-2006 |
| | | | EP | 2138129 A1 | 30-12-2009 |
| | | | ES | 2327557 T3 | 30-10-2009 |
| | | | JP | 4478369 B2 | 09-06-2010 |
| | | | JP | 2003505192 A | 12-02-2003 |
| | | | US | 6142987 A | 07-11-2000 |
| | | | US | 6652505 B1 | 25-11-2003 |
| | | | US | 2004059372 A1 | 25-03-2004 |
| | | | US | 2007219491 A1 | 20-09-2007 |
| | | | US | 2011066177 A1 | 17-03-2011 |
| | | | WO | 0108596 A1 | 08-02-2001 |
| US 2007083193 | A1 | 12-04-2007 | AU | 2006283052 A1 | 01-03-2007 |
| | | | CA | 2620080 A1 | 01-03-2007 |
| | | | EP | 1928336 A2 | 11-06-2008 |
| | | | US | 2007083193 A1 | 12-04-2007 |
| | | | US | 2014171942 A1 | 19-06-2014 |
| | | | WO | 2007024983 A2 | 01-03-2007 |
| US 5046497 | A | 10-09-1991 | NONE | | |
| US 2006167398 | A1 | 27-07-2006 | CN | 101132749 A | 27-02-2008 |
| | | | EP | 1845915 A2 | 24-10-2007 |
| | | | HK | 1117025 A1 | 28-05-2010 |
| | | | JP | 5074928 B2 | 14-11-2012 |
| | | | JP | 2008528129 A | 31-07-2008 |
| | | | US | 2006167398 A1 | 27-07-2006 |
| | | | US | 2006167399 A1 | 27-07-2006 |
| | | | US | 2010179466 A1 | 15-07-2010 |
| | | | US | 2010331755 A1 | 30-12-2010 |
| | | | WO | 2006081288 A2 | 03-08-2006 |
| US 2004193021 | A1 | 30-09-2004 | AU | 2003296956 A1 | 30-06-2004 |
| | | | CA | 2508800 A1 | 24-06-2004 |
| | | | EP | 1581102 A2 | 05-10-2005 |
| | | | JP | 2006509547 A | 23-03-2006 |
| | | | US | 2004193021 A1 | 30-09-2004 |
| | | | WO | 2004052182 A2 | 24-06-2004 |
| US 4928693 | A | 29-05-1990 | BR | 9001187 A | 19-03-1991 |

EPO FORM P0459

For more details about this annex : see Official Journal of the European Patent Office, No. 12/82

page 1 of 3

## ANNEX TO THE EUROPEAN SEARCH REPORT
## ON EUROPEAN PATENT APPLICATION NO.

EP 17 17 6629

This annex lists the patent family members relating to the patent documents cited in the above-mentioned European search report.
The members are as contained in the European Patent Office EDP file on
The European Patent Office is in no way liable for these particulars which are merely given for the purpose of information.

06-11-2017

| Patent document cited in search report | | Publication date | Patent family member(s) | | Publication date |
|---|---|---|---|---|---|
| | | | CA | 2011822 A1 | 13-09-1990 |
| | | | EP | 0388113 A2 | 19-09-1990 |
| | | | JP | H0332640 A | 13-02-1991 |
| | | | JP | H0558729 B2 | 27-08-1993 |
| | | | NO | 901158 A | 14-09-1990 |
| | | | PT | 93404 A | 08-01-1991 |
| | | | US | 4928693 A | 29-05-1990 |
| | | | ZA | 9001882 D | 30-10-1991 |
| US 2005283221 | A1 | 22-12-2005 | AT | 401926 T | 15-08-2008 |
| | | | AT | 508764 T | 15-05-2011 |
| | | | AU | 2005262753 A1 | 19-01-2006 |
| | | | CA | 2569792 A1 | 19-01-2006 |
| | | | EP | 1755722 A1 | 28-02-2007 |
| | | | EP | 1990069 A1 | 12-11-2008 |
| | | | ES | 2364465 T3 | 02-09-2011 |
| | | | JP | 5684594 B2 | 11-03-2015 |
| | | | JP | 2008503249 A | 07-02-2008 |
| | | | JP | 2011083651 A | 28-04-2011 |
| | | | JP | 2012011262 A | 19-01-2012 |
| | | | JP | 2013081864 A | 09-05-2013 |
| | | | JP | 2015006584 A | 15-01-2015 |
| | | | JP | 2015062690 A | 09-04-2015 |
| | | | US | 2005283221 A1 | 22-12-2005 |
| | | | WO | 2006007137 A1 | 19-01-2006 |
| US 2008015541 | A1 | 17-01-2008 | AT | 390154 T | 15-04-2008 |
| | | | AU | 767873 B2 | 27-11-2003 |
| | | | BR | 9910169 A | 31-12-2002 |
| | | | CA | 2329013 A1 | 11-11-1999 |
| | | | CN | 1308508 A | 15-08-2001 |
| | | | DE | 69938425 T2 | 02-01-2009 |
| | | | EP | 1079874 A2 | 07-03-2001 |
| | | | EP | 1949921 A2 | 30-07-2008 |
| | | | EP | 2335748 A1 | 22-06-2011 |
| | | | ES | 2304808 T3 | 16-10-2008 |
| | | | ES | 2430352 T3 | 20-11-2013 |
| | | | ES | 2440723 T3 | 30-01-2014 |
| | | | JP | 4219558 B2 | 04-02-2009 |
| | | | JP | 2002513646 A | 14-05-2002 |
| | | | US | 6511492 B1 | 28-01-2003 |
| | | | US | 6685722 B1 | 03-02-2004 |
| | | | US | 2004133232 A1 | 08-07-2004 |
| | | | US | 2008015541 A1 | 17-01-2008 |
| | | | US | 2010145371 A1 | 10-06-2010 |
| | | | US | 2014257245 A1 | 11-09-2014 |

EPO FORM P0459

For more details about this annex : see Official Journal of the European Patent Office, No. 12/82

page 2 of 3

**ANNEX TO THE EUROPEAN SEARCH REPORT
ON EUROPEAN PATENT APPLICATION NO.**

EP 17 17 6629

This annex lists the patent family members relating to the patent documents cited in the above-mentioned European search report.
The members are as contained in the European Patent Office EDP file on
The European Patent Office is in no way liable for these particulars which are merely given for the purpose of information.

06-11-2017

| Patent document cited in search report | Publication date | Patent family member(s) | Publication date |
|---|---|---|---|
| | | WO 9956801 A2 | 11-11-1999 |

For more details about this annex : see Official Journal of the European Patent Office, No. 12/82

EPO FORM P0459

page 3 of 3

**EP 3 260 156 A1**

**REFERENCES CITED IN THE DESCRIPTION**

**Patent documents cited in the description**

- WO 2003039626 A
- US 6409863 B
- US 6565514 B
- US 6615667 B
- US 6233476 B
- US 20040097804 A